# NEW EUROPEAN PATENT SPECIFICATION

(11) **EP 0 106 662 B2**
(45) Date of publication and mention of the opposition decision: **08.06.1994**
(45) Mention of the grant of the patent: 23.11.1988
(21) Application number: 83306178.1
(22) Date of filing: 12.10.1983
(51) Int. Cl.: G01N 21/03, B01L 3/00, G01N 33/52

(54) **Non-fluorescent vessels for holding test samples in fluorescent assays**
Nichtfluoreszierende Behälter zum Halten von Proben in einem Fluoreszenztest
Récipients non-fluorescents pour contenir des échantillons dans un test par fluorescence

(30) Priority: 12.10.1982 US 433826
(43) Date of publication of application: 25.04.1984
(73) Proprietor: DYNATECH LABORATORIES, INCORPORATED, Alexandria Virginia 22314 (US)
(72) Inventor: Nelson, Keith E., Virdes California 90274 (US); Crawford, Claude C., Annapolis Maryland 21402 (US)
(74) Representative: Warren, Keith Stanley

(56) References cited:
- EP-A-01 085 24
- DE-A- 2 617 908
- FR-A- 2 359 422
- GB-A-20 566 70
- US-A- 3 356 462
- US-A-41 547 95
- WO - A - 82/00359

## Description

This invention generally relates to laboratory equipment for use in conducting fluorescent assays and is particularly concerned with vessels for holding test samples to be measured for their fluorescence.

Fluorescent assays are now commonly used for diagnostic and research purposes to detect and measure the quantity of a wide variety of immunological and non-immunological substances. In fluorescent immunoassays, the test sample may be prepared for fluoromatic measurement in a variety of different ways.

For example, a fluorescently labeled reactant may be immunologically reacted with the immunological substance of interest which is usually directly or indirectly immobilized on a solid phase. Following separation of the nonspecific substances which are not attached to the substance of interest, the fluorescence of the reaction product is measured to determine the amount of the substance of interest. In another type of fluorescent immunoassay, an enzyme labeled reactant is attached to the immunological substance of interest, and a fluorogenic substrate is catalyzed by the enzyme label to yield a fluorescent product which can be fluorescently measured to determine the quantity of the substance of interest.

In carrying out fluorescent assays of the foregoing type and also other types, microtest plates (or mi- crotitration plates, as they are also called) and strips of microtest wells are often used. Microtest plates are formed with a multiplicity of wells which are joined together in a molded one-piece structure for containing microliter quantities of fluid samples. Examples of a microtest plate and microtest wells in strip form are described in U.S. Patent No. 4,154,795 which issued to A. C. Thorne on May 15, 1979.

The use of microtest plates and microtest strips of wells in fluorescent and other types of assays offers several important advantages. First. they permit the mass preparation of a large number of test sample solutions at the same time. Second, they are more convenient to handle as compared with individual test tubes. Third, they can easily and inexpensively be washed. Fourth, they are inexpensive and disposable. Fifth, they are customarily formed from plastic materials which are not fragile like glass. Sixth, they can be made from a material having an ability to attract certain molecules such as protein molecules so that they can serve as a solid phase in an immunoassay. Polystyrene and polyvinyl chloride are commonly used for this purpose and exhibit acceptable protein binding properties for attracting protein molecules.

The use of plastic materials permits the microtest plates and strips to be manufactured by low cost, mass production molding techniques.

For fluorescent assays, however, molded microtest plates and strips of the type described above have a serious drawback in that the moldable materials customarily used for low cost manufacture exhibit a substantial level of native fluorescence, particularly at the exciting light wave lengths commonly employed in fluorometers. When used to hold a testsam- ple in a fluorometer for making a fluorometric measurement they therefore will unavoidably be excited along with the test sample by the fluorometer's exciting light. As a result, the microtest plates, strips or their individual wells will fluoresce to produce spurious light emissions which interfere with and impair an accurate measurement of the intensity of the light emitted from the excited test sample itself. These Spurious light emissions have the objectionable effect of creating a noise signal in the fluorometer's de- tectorto significantly reduce the signal-to-noise ratio.

One solution to the foregoing problem is to equip the fluorometers with special, sample-holding vessels made of non-fluorescent or low-fluorescing materials such as quartz or certain kinds of glass. In addition, optical grade Teflon has been suggested as a non-fluorescing material for making fluorometer flow cells in U.S. Patent No. 4,008,397 which issued to J. J. Zarodowski on February 15. 1977. also, an Italian company called Kartell is marketing a molded cuvette of undisclosed plastic material which is claimed to have a sufficiently low level of fluorescence to make it suitable for holding samples in a fluorometer. Although the level of fluorescence of the Kartell cuvette is lower than that of clear polystyrene, it nevertheless is significant.

The foregoing cuvettes and test tubes share a common drawback in that they are each capable of holding only a single test sample. Vessels of this type are therefore less convenient to work with as compared with microtest plates or strips. More particularly, they are not suitable for the mass preparation of test samples as with microtest plates and strips. In addition, they are more difficult and more expensive to wash in the course of performing the various steps in an immunoassay.

Furthermore, quartz and glass are not suitable for making the intricately shaped microtest plates and strips because the manufacturing costs would be prohibitively high. In addition, the protein binding properties of quartz and glass are inferior to the protein binding properties of polystyrene and other plastics which are customarily used for making microtest plates and strips. They therefore are not as suitable as polystyrene and other plastics for defining a solid phase in an immunoassay. Finally, they are fragile and are not intended to be disposable.

Because of the disadvantages associated with cuvettes or tubes, it is often desirable and sometimes necessary to prepare the test samples in a standard microtest plate or strip and then to transfer the samples to the cuvettes or tubes for measurement. Transferring the test samples is time consuming, inconvenient and increases the cost of the fluorescent assay.

From the foregoing, it is clear that, on the one hand, there are easily moldable materials (such as polystyrene and polyvinyl chloride) which are suitable for use in low cost, mass production of microtest plates and other sample-holding vessels, but which exhibt a substantial level of native fluorescence to create a problem in measuring the fluorescence of test samples. On the other hand, there are materials such as quartz and certain kinds of glass which have low levels of native fluorescence to avoid the foregoing problem, but which are unsuitable for the low cost production of microtest plates and strips.

With the foregoing in mind, the general aim and purpose of this invention is to provide a novel, low cost, sample-holding vessel in which the native fluorescence of the material used to form the vessel is effectively suppressed or reduced to enhance the fluorometer's signal-to-noise ratio in a fluorometric measurement.

By suppressing the vessel's native fluorescence, virtually any desired material may be used for forming the vessel, including those which are used to make present day microtest plates and strips. With this invention, microtest plates and strips may therefore be manufactured with their customary materials (such as polystyrene or polyvinyl chloride) at low, affordable costs without encountering the problem associated with the materials native fluorescence. Vessels made in accordance with this invention are therefore suitable both for preparing the test sample and for holding the prepared test sample during the fluorometric measurement.

The foregoing object is achieved by providing the vessel with a barrier which blocks any significant penetration of the fluorometer's exciting light into the body of the sample-holding vessel, thereby preventing the vessel's material from being excited during the fluorometric measurement. When exposed to the fluorometer's exciting light, therefore, the vessel itself will not emit any significant light to interfere with the fluorometric measurement of the light emissions from the excited test sample. The signal-to-noise ratio in the fluorometer's detector is therefore improved to improve the accuracy of the fluorometric measurement.

In the illustrated embodiment 5, the barrier is of the physical type which is non-chemically incorporated with the vessel's material and which therefore does not alter the chemical properties of the vessel's material. The barrier is formed by dispersing pigment particles throughout the body of the vessel, which pigment particles are opaque or substantially opaque to the wave length of the fluorometer's exciting light source and are non-fluorescent or at least exhibit low fluorescent at the wave length of the light emitted from the excited test sample.

Black pigment is especially suitable for forming the barrier in the sample-holding vessel of this invention. It is opaque to and absorbs all lightwave lengths. A plastic vessel containing a black pigment barrier exhibits virtually no detectable light emissions when subjected to light in the ultraviolet range.

White pigment is also highly suitable for forming the barrier in the sample-holding vessel of this invention. While a vessel containing a white pigment barrier wi fluoresce to some extent when exposed to ultraviolet light, the white pigment has the effect of appreciably strengthening the light emission produced by exciting the test sample, thereby improving the signal-to-noise ratio by both strengthening the signal of interest (the wave length of the light emitted by the excited test sample) and reducing the objectionable noise signal (the spurious light emission from the vessel itself).

Apart from white and black pigments, pigments of certain colors may also be suitable as long as they are opaque to the wave lengths of the fluorometer's exciting light and are non-fluorescent or exhibit low flourescence at the wave length of the light emitted by the excited test sample.

It will be appreciated that the vessel incorporating the principles of this invention is advantageously molded by low cost mass production techniques.

In order that the invention may be more readily understood, reference will now be made to the accompanying drawings, in which:-
Figure 1 is a perspective view of a 96 well microtest plate incorporating the principles of this invention;
Figure 2 is an enlarged, fragmentary section taken substantially along lines 2-2 of Figure 1;
Figure 3 is a perspective view of a microtest strip of wells;
Figure 4 is an enlarged fragmentary section taken substantially along lines 5-5 of Figure 3 and
Figure 5 is a schematic view of a fluorometer which is adapted for use with the sample-holding vessels of this invention;

For convenience, the term "vessel" is used in this specification (including the claims) to mean microtest plates, strips of wells, or any other microtest well structure having a plurality of open-top wells for holding liquid. Also, the term "light" as used in this specification refers to both non-visible light (e.g., ultraviolet light) and light which is visible to the naked eye.

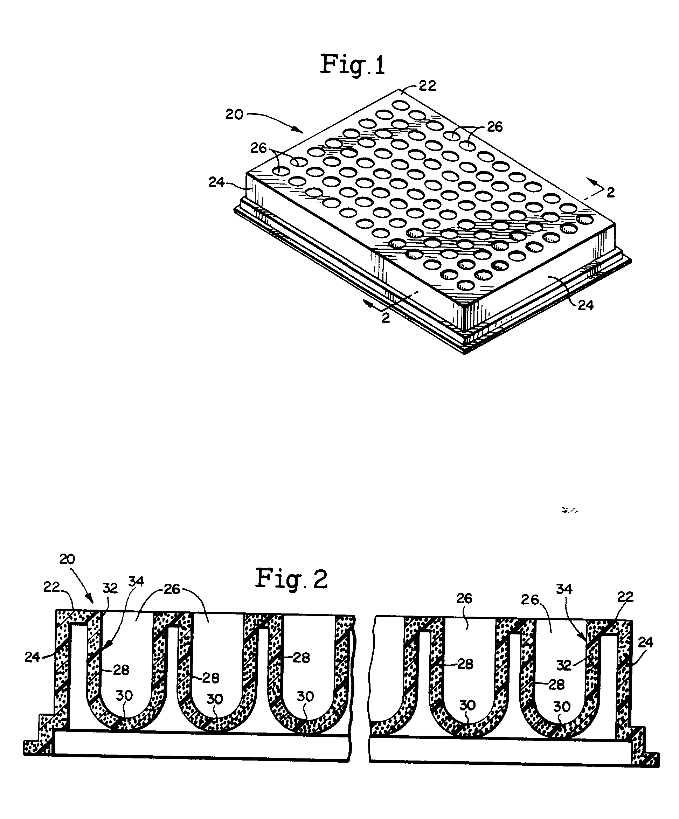

Referring to Figures 1 and 2, a molded, one-piece, rectangular microtest plate 20 incorporating the principles of this invention is integrally formed with a flat top wall 22 and side walls 24 depending from top wall 22 to form a depending skirt along all four sides of the plate to support the plate on a flat horizontal surface. Plate 20 is additionally formed with a multiplicity of precisely dimensioned, upwardly opening wells (or cups as they are also called) 26 for holding microliter quantities of liquid test samples or other solutions.

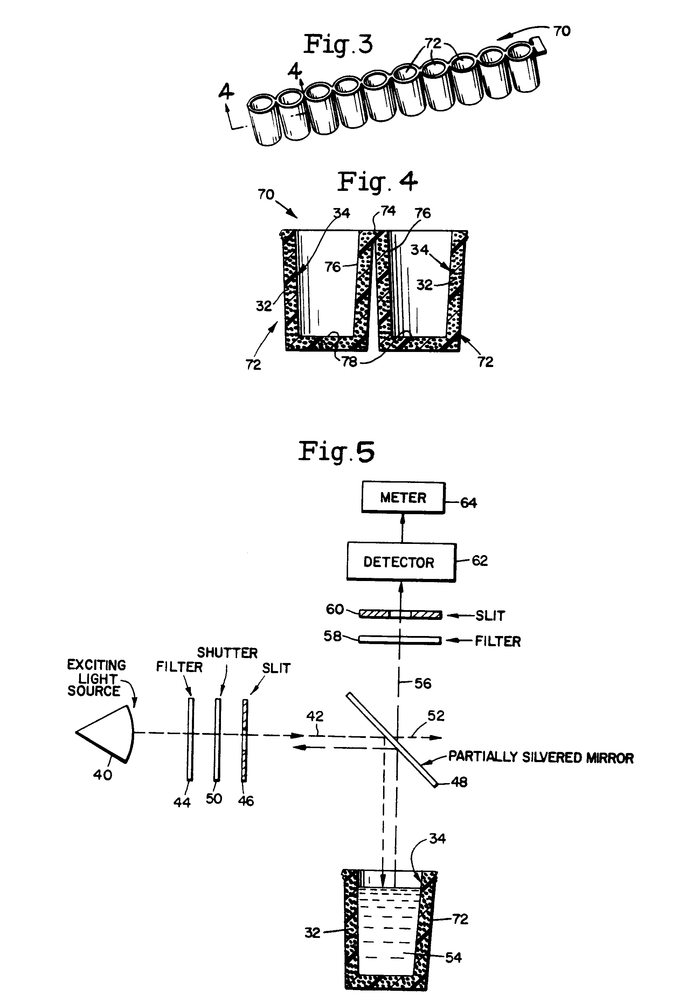

Wells 26 are integral with and open at top wall 22. By this construction, wells 26 are integrally joined to each other through top wall 22 and, unlike the embodiment shown in Figure 3, are not intended to be separated from each other.

As best shown in Figure 2, wells 26 depend from top wall 22 to lie in the region delimited by side walls 24. Wells 26 are uniformly spaced apart from each other and are uniformly dimensioned. Each of the wells 26 is formed with a cylindrical side wall 28 and a suitable bottom 30. The thicknesses of the wells' side and bottom walls are essentially uniform and are relatively small. The longitudinal axes of wells 26 are parallel and normally intersect top wall 22. In the embodiment illustrated in Figures 1 and 2, there are twelve parallel spaced apart rows of wells with eight wells in each row to provide the standard total of 96 wells.

The foregoing type of microtest plate is essentially the same as the one described in U.S. Patent No. 3,356,462 which issued to N. M. Cooke et al on December 5, 1967.

Plate 20 is preferably formed from commercially available polystyrene or polyvinyl chloride for use in immunoassays. For example, the polystyrene may be any one of the following: Gulf SMD 3500, Foster Grant 9100D, Monsanto Lustrex 777 or Dow Chemical clear general purpose 666U. Plate 20 may alternatively be formed from any one of a number of other moldable materials such as acrylonitrile-butadienestyrene and related multipolymers, acetal and homopolymer and copolymers thereof (Delrin forexample), acrylics such as Plexiglass, allyl esters, amino resins, cellulosic plastics, epoxies, certain fluoroplastics, furan resin, ionomers, nitrile resins, phenolics, modified phenylene oxide, polyamide (nylon), polyamideimide, polybutylene, polycarbonate (Lexan for example), polyester derivatives, polyethylene and ethylene copolymers, ethylene-vinyl acetate, polyimide, polymethylpentene, polyphenylene sulfide, polypropylene, derivatives of polystyrene, polyurethane, vinyl and polyvinyl copolymers other than polyvinyl chloride, silicone, styrene-acrylonitrile, sulfone polymers, vinylidene chloride and polymers and copolymers thereof, and alloys of the above.

The plastic material selected for plate 20 is required to be chemically compatible with the assay substances to be used so as not to upset or interfere with the reactions which take place in assays, particularly immunoassays. In general, the particular application (e.g., immunoassays) determines the material to be used for making plate 20.

In accordance with this invention, pigment particles 32 are uniformly distributed or dispersed throughout plate 20 in sufficient quantity or density to form a barrier 34 which blocks any significant penetration of a fluorometer's exciting light or irradiation into plate 20, thereby preventing any significant excitation of the plate's plastic material during a fluorometric measurement. The pigment-defining barrier therefore has the effect of suppressing the native fluorescence of the plate's plastic material.

It will be appreciated that only the layer of pigment 32 lying immediately adjacent to the exposed surfaces of plate 20 defines the barrier for blocking penetration of the fluorometer's exciting light into plate 20. At best, therefore, only the plastic molecules lying on the plate's exposed surfaces will be fluoresced when exposed to a fluorometer's exciting light. The number of plastic molecules which will be fluoresced will consequently be reduced to a negligible number.

Pigment particles 32 are required to be opaque or at least substantially opaque at least to the wave length range of the fluorometer's exciting light. In addition, pigment particles 32 are required to be non-fluorescent or at east exhibit low fluorescence (which is less than that of the plastic material used to form plate 20) at the wave length of the light emissions resulting from excitation or irradiation of the test samples in wells 26.

Black and white pigments are especially suitable for defining the desired barrier previously mentioned. Other colored pigments such as green and red may also be used for defining barrier 34 for blocking penetration of the fluorometer's exciting light, so long as they are opaque at least to the wave length range of the fluorometer's exciting light and are non-fluorescent or at least exhibit low fluorescence at the wave lengths of the light emissions which result from excitation of the test samples.

A colored pigment other than white wi ll reflect the particular wave length of its color so that the selection of a colored pigment other than white will afford selective enhancement of certain light wave lengths. For example, a red pigment will reflect the red wave length while absorbing all other wave lengths in the light spectrum.

Black pigment, of course, absorbs all wave lengths in the visible and non-visible light spectrum and will therefore reflect none, while white pigment reflects all wave lengths in the spectrum, while absorbing none. It will be appreciated that the reflection of light produced by white pigment or other colored pigments differs from, and therefore should be distinguished from, light emissions originating from the native fluorescence of the material due to irradiation or excitation by an exciting light or radiation, which is usually in the ultraviolet range.

The pigment selected for establishing barrier 34 in plate 20 is required to be chemically compatible with the substances to be placed in wells 26 so that it does not alter the substances themselves or upset the reactions in assays which are carried out using plate 20. It will be appreciated that pigment particles 32 are merely mixed with the plastic material of plate 20 and therefore are not chemically combined with the plate's plastic material. Pigment particles 32 therefore do not alter the chemical properties of the plate's plastic material.

In order to form barrier 34, it is not necessary that pigment particles 32 be uniformly dispersed throughout plate 20. Instead, they need only be dispersed to an adequate extent to form the desired barrier for blocking penetration of a fluorometer's exciting light into the plate.

From the foregoing description it is evident that the amount of pigment particles 32 incorporated into plate 20 must be sufficient to prevent any significant penetration of a fluorometer's exciting light into the microtest plate to thereby suppress or at least reduce the native fluoroscence of the plastic material used to form plate 20. On the other hand, the amount of pigment particles 32 used to form barrier 34 should not be so great as to compromise or impairthe structural strength of plate 20 or to upset or adversely affect the molding conditions for molding plate 20 from a selected plastic.

In carrying out an embodiment of this invention, pigment particles 32 are added to and thoroughly mixed with the plastic material prior to molding of the plate, so that barrier 34 is effectively formed in situ in the course of molding plate 20 into its final form. A satisfactory barrier is achieved by adding pigment to the selected plastic material in an amount ranging from about 0.01% to about 10% based on the total weight of the plate's materials (i.e, the weight of the plastic material plus the weight of the pigment particles). Percentages of approximately 0.1% to approximately 1.0%, however, are preferred. The minimum amount of pigment particles will vary depending upon the type of pigment selected and also on the particular applications which plate 20 will be used for. The limit on the maximum amount of pigment will, as previously noted, depend primarily on the desired structural strength of plate 20 or, for that matter, any other vessel which is formed with the pigment-defining barrier of this invention.

In one preparation for the microtest plate for use in fluorescent immunoassays, commercially available carbon based black pigment (PMS 4500 SUDC, which is pure carbon and which is supplied by Plastic Molding Supply, a company located in Brunswick, New Jersey) was mixed with commercially available clear polystyrene in an amount equal or proportional to 0.22 pounds (100 grams) of the black carbon pigment per 100 pounds of clear polystyrene. The amount of pigment present in the mixture therefore amounted to slightly less than 0.22% of the total weight of the mixture or exactly 0.22% of the weight of the total weight of the polystyrene. Following mixing to uniformly disperse or distribute the pigment throughout the plastic, microtest plates of the type shown in Figure 1 were injection molded from the pigment-plastic mixture. Microtest plates produced from this mixture were observed to have a black, opaque appearance.

When exposed to an exciting light in the ultraviolet range, plates made from the foregoing black pigment-polystyrene formulation exhibited no detectable light emissions or, at best, only negligible light emissions when exposed to an exciting light in the ultraviolet range. Accordingly, the black-pigmented microtest plates described in the foregoing example have the effect of dramatically increasing the signal-to-noise ratio of a fluorometer's light intensity detector.

In another example, commercially available white pigment (Plastic Molding Supply's PMS 350 SUDC) was mixed with the same polystyrene used in the preceding example in the amounts specified in the first example for black pigment (i.e., 0.22 lbs. of white pigment per 100 lbs. of polystyrene). Microtest plates injection molded from the white-pigmented mixture were found to reduce the intensity of the vessel's fluorscently produced light emissions to almost one-half the value measured for conventional clear polystyrene microtest plates without the pigment-defining barrier of this invention.

Although not reducing the light emissions due to native fluorescence as much as black pigment, the white pigment was found to have the advantageous effect of substantially increasing the intensity of the light emissions resulting from the fluorescent excitation of test samples in the plate's wells, thereby strengthening the signal of interest due to the light emitted by the excited test samples. Accordingly, white pigment has the effect of improving the signal-to-noise ratio in two ways, one by strengthening the signal of interest, and the other by reducing the noise signal due to the plate's native fluorescence. The increase in the intensity of the light emitted by the fluorescently excited test sample in the white-pigmented microtest plate is believed to be due to the high reflection property of white pigment which causes the fluorometer's exciting light and also the emitted light (due to the fluorescent excitation of the test sample) to be reflected from the surfaces of the sample-receiving wells 26 in the microtest plate. This reflection appears to cause the exciting light and emitted light to move or bounce back and forth through the sample to increase the intensity of the light emitted by the fluorescently excited sample.

Another white pigment considered to be suitable for forming barrier 34 is Dupont's Ti-Pure R-101 CFKD, which is titanium dioxide. It is evident that any other type of white or black pigment may be used so long as it is opaque or at least substantially opaque to the wave length of the fluorometer's exciting light and is non-fluorescent or at least exhibits low fluorescence at the wave length of the light emitted by the fluorescently excited test sample.

The exciting light in the embodiment of Figures 1 and 2 is directed through the open end of each of the plate's wells. To conduct a fluorometric measurement it therefore will be appreciated that a fluorometer of the frontal approach type is required such as the one schematically shown in Figure 5.

In Figure 5, the fluorometer comprises a suitable light source or source of radiation 40 for supplying an exciting light or radiation which may vary from about 200 nanometers to about 700 nanometers depending upon the material to be fluoresced and which is usually in the ultraviolet wave length range.

The exciting light beam developed by source 40 is indicated by arrow 42 and is directed through a filter 44 and a silt or aperture 46 to a partially silvered mirror48. Mirror48 is located at an acute angle to the direction of the exciting light from source 40 to reflect the exciting light beam downwardly through the open upper end of a sample holding well so that it impinges directly on and penetrates the test sample in the well before impinging any of the well's surfaces. A shutter 50 may optionally be located between filter44 and slit 48. The beam of exciting light 42 may be focused or suitably targeted so that upon being reflected by mirror48 itwill enter the well through the well's open upper end. Because mirror 48 is only partially silvered, part of the exciting light will pass through the mirror and will therefore be wasted as indicated by the arrow 52.

Upon being exposed to the exciting light, the test sample (indicated at 54 in Figure 7) will be fluorescently excited to emit light at a predetermined wave length or range of wave lengths. The wave length of the emitted light depends on the particular substance which is fluorescently excited and is typically in the range extending from about 350 nm to about 900 nm. The emitted light produced by the excitation of test sample 54 will pass directly upwardly through the open upper end of the well and will pass through the partially silvered mirror 48 as indicated by arrow 56. Part of the emitted light will be reflected by the partially silvered mirror and will therefore be wasted.

The emitted light 56 passing through mirror 48 will pass through a further filter 58 (which may be of the bandpass type to pass just the wave length of interest) and another slit or aperture 60 to a detector 62 which measures the intensity of the emitted light. A device such as a meter 64 is connected to detector 62 and supplies a read-out of the intensity of the emitted light detected in detector 62 to provide a fluorometric measurement of the intensity of the emitted light and, therefore, the fluorescence of the fluorescently excited substance in test sample 54.

Another frontal approach type of fluorometer which is suitable for measuring the fluorescence of samples in wells 26, is described in EP-A-0 108 524.

From the foregoing description it will be appreciated that the beam of exciting light 42 enters trough the open upper end of the sample-holding well, and that the light emission due to the fluorescent excitation of sample 54 exits through the open upper end of the well. The provision of barrier 34 in the form shown in Figure 1 or in the form shown in Figure 3 therefore does not interfere with the entrance of exciting light or the exit or emitted light.

It is evident from the foregoing that any suitable frontal approach type of fluorometer may be used with sample-holding vessels containing the barrier of this invention. The particular type of frontal approach fluorometer therefore does not constitute a part of this invention.

In Figures 3 and 4 the pigment-defining barrier of this invention is shown to be incorporated into a molded one-piece microtest strip 70 containing a straight row of parallel, upwardly opening wells 72 for containing microliter quantities of liquid. In this embodiment, each of the wells 72 is formed with a cylindrical side wall 76 and a flat bottom wall 78 as shown in Figure 4.

Each of the wells 70 is integrally joined to the adjacently disposed wells by frangible stem-like segments 74 which may take the form of mold flashings. Segments 74 lie flush with the lips or open upper ends of wells 72. Segments 74 are easily broken by hand to enable one or more of the wells 72 to be separated from the strip. One of the disconnected wells 72 is shown in Figure 5.

Wells 72 are provided with precise, uniform dimensions. The longitudinal axes of wells are parallel and contained in a common plane.

The previously mentioned Thorne patent US-A-4 154 795 discloses a matrix of wefts which are integrally joined together by frangible stems for separation into individual wells and also into strips of the type shown in Figure 3. It will be appreciated that the principles of this invention may be applied to the well structure described in US-A-4 154 795.

Strip 70 is preferably formed from polystyrene or polyvinyl chloride. Alternatively, strip 70 may be formed from any of the other moldable materials mentioned for plates 20.

Where strip 70 is formed from polyvinyl chloride, the individual wells 72 may be integrally joined together by a thin severable segment portion (not shown) which can easily be cut with scissors.

A holder of the type shown and described in the Thorne patent may be used to support strip 70 and/or the individual wells. Alternatively, any other suitable holder may be used to support the strip while conducting an assay. If desired the holder itself may be provided with the barrier of this invention.

Like the embodiment shown in Figure 2, pigment particles 32 are uniformly distributed or dispersed throughout strip 70 in sufficient quantity or density to form the required barrier 34 which blocks any significant penetration of the fluorometer's exciting light or radiation into strip 70, thereby preventing any significant excitation of the strip's plastic material during a fluorometric measurement. The pigment-defining barrier for strip 70 therefore has the effect of supress- ing the native fluorescence of the strip's plastic material in the same manner described for the embodiment of Figure 2.

The pigments used to form the barrier in the embodiment of Figure 2 are intended for use to form the barrier in strip 70. Like Figure 2, black or white pigment is especially suitable for forming the barrier in strip 70. The amount of pigment used to form the barrier in strip 70 is the same as that described for the embodiment in Figure 2.

In manufacturing strip 70, it is evident that pigment particles 32 are first thoroughly mixed with the plastic material prior to molding strip 70. Thereafter, strip 70 is molded from the mixture of the plastic material and pigment particles so that the pigment particles are captured or suspended in the solidified, molded plastic body to define the barrier 34.

The vessels incorporating the barrier of this invention are molded. Any suitable conventional molding technique may be utilized, such as injection molding, thermoforming and casting. Injection molding is commonly used for manufacturing polystyrene microtest plates, strips and other vessels. For manufacturing polyvinyl chloride microtest plates, strips and other vessels, the barrier-defining pigment is first mixed thoroughly with the plastic resin, and the mixture is then extruded into a sheet. Thereafter, the sheet is thermoformed into the microtest plates, strips and other vessels.

From the foregoing description, it is clear that the microtest plates, strips and other vessels incorporating the barrier of this invention, may be placed directly in a fluorometer of the type shown in Figure 5 for measuring the fluorescence of a test sample in wells 26, 72. Where microtest plates 20 are inserted into the fluorometer, it is understood that either the plate orthe fluorometer's exciting light beam must be shifted to separately measure the flourescence of each sample. Any conventional carrier (not shown) may be used for shifting the microtest plate in an X-Y plane to target each well separately. The same applies where the microtest strip or a portion of the microtest strip is inserted into the fluorometer.

In conducting a fluorescent immunoassay with the microtest plates or microtest strips described above, it is evident that the test samples are first prepared in the sample-receiving wells. Thereafter, the entire microtest plate, the entire microtest strip or, the individual wells (as in the case of the microtest strip) is placed in the fluorometer for conducting the fluorometric measurement of the test sample. The measurement of the test sample's fluorescence is then used to determine the quantity of the substance of interest in the test sample as outlined in the introductory portion of this specification.

## Claims

1. A microtest well structure (20, 70) having a plurality of integral open top wells (26, 72) for receiving and holding microliter quantities of fluid samples during a fluorometric measurement in which each of the samples is exposed to an exciting radiation to be fluorescently excited thereby, said wells (26, 72) being arranged in at least one straight row and being formed as a one-piece molding from a plastics material having a native fluorescence, and means (32) forming a barrier (34) which prevents radiation and/or light directed at the walls (28, 30, 76, 78) of the wells from passing therethrough, characterised in that the barrierforming means comprises pigment particles which are dispersed throughout the molding and which block or at least reduce the extent of penetration of the exciting radiation into the molding at least to reduce the extent to which the plastics material is fluorescently excited by the exciting radiation during said measurement where the exciting radiation is directed to pass into each well through the open top thereof, said pigment particles being present in an amount ranging from 0.01% to 10% based on the combined weight of plastics material plus the weight of the pigment particles and being at least substantially opaque at least to the peak wavelength of the exciting radiation, and said pigment being substantially non-fluorescent.

2. A microtest well structure according to claim 1, characterised in that the pigment particles are black or white.

3. A microtest well structure for conducting assays of immunological substances in a frontal approach-type energy measurement instrument, wherein a labelled reactant is immunologically reacted with an immuno-logical substance which is directly or indirectly immobilized on a solid phase to yield a light-emitting reaction product wherein the energy release of said reaction product is measured to determine the quantity of the substance, said structure (20, 70) having a plurality of open-top wells (26, 72) for receiving and holding microliter quantities of fluid samples during a measurement, said wells being molded from a plastics material having a native fluorescence and sufficient protein-binding properties to serve as the solid phase in said assay, and barrier means (32) which prevents radiation and/or light directed at the walls (28, 30, 76, 78) from passing therethrough, characterised in that the barrier means (32) is substantially opaque and substantially non-fluorescent and is dispersed throughout the plastics material for enhancing the signal-to-noise ratio of the system during said measurement, said barrier means comprising pigment particles dispersed in those regions in an amount ranging from 0.01% to 10% based on the combined weight of plastics material plus the weight of the pigment particles.

4. A microtest well structure according to claim 3, characterised in that pigment particles (32) act to block or at least reduce the extent to which said plastics material is fluorescently excited to thereby enhance the signal-to-noise ratio of said system during said measurement.

5. A microtest well structure according to claim 3, characterised in that the pigment particles (32) act to collect and reflect the energy of said reaction product thereby to enhance the signal-to-noise ratio of said system during said measurement.

6. A microtest well structure according to claim 4, characterised in that the pigment particles are black.

7. A microtest well structure according to claim 5, characterised in that the pigment particles are white.

8. A microtest well structure according to any preceding claim, characterised in that the plastics material comprises polystyrene or polyvinyl chloride.

## Patentansprüche

1. Mikrotestbehälteraufbau (20, 70) mit einer Vielzahl von eingearbeiteten, nach oben hin offenen Behältern (26, 72) zum Sammeln und zurAufnahme von Mikrolitermengen von Flüssigkeitsproben während einer fluorometrischen Messung, bei der jede der Proben einer anregenden Strahlung ausgesetzt wird, um dadurch fluoreszierend angeregt zu werden, wobei die Behälter (26, 72) wenigstens in einer geraden Reihe angeordnet und als einstückiges Formteil aus Kunststoff mit einer natürlichen Fluoreszenz ausgebildet sind, und mit eine Schranke (34) bildenden Mitteln (32), welche das Durchdringen von Strahlung und/oder Licht, die bzw. das auf die Wände (28, 30, 76, 78) der Behälter gerichtet ist, durch dieselben verhindert, dadurch gekennzeichnet, daß die eine Schranke bildenden Mittel Pigmentpartikel aufweisen, die über das Formteil hindurch verstreut sind und die den Grad des Durchdringens der anregenden Strahlung in das Formteil hinein blockieren oder wenigstens reduzieren, um zumindest den Grad der Fluoreszenz des Kunststoffes, zu der dieser durch die anregende Strahlung während der Messung, bei der die anregende Strahlung so gerichtet ist, daß sie durch die oben liegende Öffnung desselben in diesen gelangt, angeregt wird, zu vermindern, wobei besagte Pigmentpartikel in einer Menge vorhanden sind, die sich auf 0,01 % bis 10 % beläuft, basierend auf dem kombinierten Gewicht des Kunststoffes zuzüglich dem Gewicht der Pigmentpartikel, welche zumindest im wesentlichen bis wenigstens zu der Maximalwellenlänge der anregenden Strahlung opak sind und wobei besagtes Pigment i m wesentlichen nicht fluoreszierend ist.

2. Mikrotestbehälteraufbau nach Anspruch 1, dadurch gekennzeichnet, daß die Pigmentpartikel schwarz oder weiß sind.

3. Mikrotestbehälteraufbau zum Durchführen von Untersuchungen immunologischer Substanzen in einem Frontal-Annäherungs-Energiemeßgerät, bei dem ein markiertes Reaktionsmittel immunologisch mit einer immunologischen Substanz zur Reaktion gebracht wird, die direkt oder indirektaufeinerfesten Phase immobilisiertwird, um ein lichtemittierendes Reaktionsprodukt zu ergeben, wobei die freiwerdende Energie des Reaktionsproduktes gemessen wird, um die Menge der Substanz zu bestimmen, wobei der genannte Aufbau (20, 70) eine Vielzahl von nach oben offenen Behältern (26, 72) zum Sammeln und zur Aufnahme von Mikrolitermengen von Flüssigkeitsproben während einer Messung aufweist, wobei die Behälter aus Kunststoff mit einer natürlichen Fluoreszenz und ausreichenden proteinbindenden Eigenschaften, um als feste Phase in genannten Untersuchungen zu dienen, gebildet sind, und mit eine Schranke bildenden Mitteln (32), welche das Durchdringen von Strahlung und/oder Licht, die bzw. das auf die Wände (28, 30, 76, 78) der Behälter gerichtet ist, durch dieselben verhindern, dadurch gekennzeichnet, daß die eine Schranke bildenden Mittel (32) im wesentlichen opak sind und im wesentlichen nicht fluoreszierend sind und in dem plastischen Werkstoff verstreut sind, um den Rauschabstand des Systems während der besagten Messung zu erhöhen, wobei die Schranke bildenden Mittel Pigmentpartikel aufweisen, die in diesen Bereichen in einer Menge von 0,01 % bis 10 % verstreut werden, basierend auf dem kombinierten Gewicht des Kunststoffes zuzüglich dem Gewicht der Pigmentpartikel.

4. Mikrotestbehälteraufbau nach Anspruch 3, dadurch gekennzeichnet, daß Pigmentpartikel (32) blockieren oder wenigstens den Grad vermindern, zu dem der Kunststofffluoreszierend angeregt wird, um den Rauschabstand des Systems während der besagten Messung zu erhöhen.

5. Mikrotestbehälteraufbau nach Anspruch 3, dadurch gekennzeichnet, daß die Pigmentpartikel (32) die Energie der genannten Reaktion sammeln und reflektieren, um dadurch den Rauschabstand des Systems während der Messung zu erhöhen.

6. Mikrotestbehälteraufbau nach Anspruch 4, dadurch gekennzeichnet, daß die Pigmentpartikel schwarz sind.

7. Mikrotestbehälteraufbau nach Anspruch 5, dadurch gekennzeichnet, daß die Pigmentpartikel weiß sind.

8. Mikrotestbehälteraufbau nach einem der vorhergehenden Ansprüche, dadurch gekennzeichnet, daß der Kunststoff Polystyrol oder Polyvinylchlorid aufweist.

## Revendications

1. Structure (20, 70) de réservoir de microtests comportant plusieurs réservoirs (26, 72) unitaire- ment ouverts sur le dessus pour recevoir et contenir des quantités de l'ordre du microlitre d'échantillons fluides lors d'une mesure fluoro- métrique dans laquelle chaque échantillon est exposé à un rayonnement d'excitation en vue d'exciter sa fluorescence, lesdits réservoirs (26, 72) étant disposés selon au moins une rangée droite et étant moulés d'une pièce dans une matière plastique ayant une fluorescence naturelle, et des moyens (32) formant une barrière (34) qui empêche le rayonnement et/ou la lumière dirigée sur les parois (28, 30, 76, 78) des réservoirs de passer au travers, caractérisée en ce que les moyens formant barrière comprennent des particules de pigment qui sont dispersées au sein du moule et qui arrêtent ou au moins réduisent l'importance de la pénétration du rayonnement d'excitation à l'intérieur du moule au moins pour réduire l'importance de l'excitation par fluorescence de la matière plastique par le rayonnement d'excitation pendant ladite mesure au cours de laquelle le rayonnement d'excitation estdirigé, de façon à pénétrer dans chaque réservoir par son ouverture supérieure, lesdites particules de pigment étant présentes dans une proportion de l'ordre de 0,01 % à 10% par rapport au poids combiné de la matière plastique plus le poids des par- tricules de pigment et étant au moins essentiellement opaque au moins vis-à-vis de la longueur d'onde de pic du rayonnement d'excitation, et ledit pigment étant essentiellement non-fluorescent.

2. Structure de réservoir de microtests selon la revendication 1, caractérisée en ce que les particules de pigment sont noires ou blanches.

3. Structure de réservoir de microtests pour réaliser des tests de substances immunologiques dans un appareil de mesure d'énergie du type approche frontale, dans laquelle un réactif marqué réagit de façon immunologique avec une substance immunologique qui est directement ou indirectement immobilisée sur une phase solide afin de donner un produit de réaction émetteur de lumière dans lequel la libération d'énergie dudit produit de réaction est mesuré pour en déduire la quantité de substance, ladite structure (20, 72) ayant plusieurs réservoirs (26, 72) ouverts sur le dessus afin de recevoir et de contenir des quantités de l'ordre du microlitre d'échantillons fluides lors d'une mesure, lesdits réservoirs étant moulés en une matière plastique présentant une flu- rorescence naturelle et suffisamment de propriétés de fixation de protéines pour faire office de phase solide dans lesdits tests et des moyens de barrière (32) qui empêchent le rayonnement et/ou la lumière dirigée sur les parois (28, 30, 70, 76) de passer au travers, caractérisée en ce que les moyens de barrière (32) sont sensiblement opaques et sensiblement non-fluorescents et sont dispersées au sein de la matière plastique pour accroître le rapport signal sur bruit du système pendant ladite mesure, lesdits moyens de barrière comprenant des particules de pigment dispersés dans ces zones dans une proportion de l'ordre de 0,01 % à 10% par rapport au poids combiné de la matière plastique plus le poids des particules de pigment.

4. Structure de réservoir de microtests selon la revendication 3, caractérisée en ce que les particules de pigment (32) agissent pour arrêter ou au moins réduire l'importance de l'excitation par fluorescence de ladite matière plastique de façon à accroître le rapport signal sur bruit dudit système pendant ladite mesure.

5. Structure de réservoir de microtests selon la revendication 3, caractérisée en ce que les particules de pigment (32) agissent pour collecter et ré- flechir l'énergie dudit produit de réaction pour accroître ainsi le rapport signal sur bruit dudit système pendant ladite mesure.

6. Structure de réservoir de microtests selon la revendication 4, caractérisée en ce que les particules de pigment sont noires.

7. Structure de réservoir de microtests selon la revendication 5, caractérisée en ce que les particules de pigment sont blanches.

8. Structure de réservoir de microtests selon l'une quelconque des revendications précédentes, caractérisée en ce que la matière plastique comprend du chlorure de polystyrène ou de po- lyvinyle.
